# EUROPEAN PATENT SPECIFICATION

(11) **EP 2 004 073 B1**
(45) Date of publication and mention of the grant of the patent: **17.03.2010**
(21) Application number: 07734301.0
(22) Date of filing: 03.04.2007
(51) Int. Cl.: A61B 17/70, A61B 17/82

(54) **DEVICE FOR TREATING VERTEBRAE, INCLUDING AN INTERSPINOUS IMPLANT**
VORRICHTUNG ZUR BEHANDLUNG VON WIRBELN, EINSCHLIESSLICH INTERSPINÖSES IMPLANTAT
APPAREIL POUR TRAITER LES VERTÈBRES, COMPRENANT UN IMPLANT INTERÉPINEUX

(30) Priority: 13.04.2006 FR 0603266; 14.04.2006 US 791906 P
(43) Date of publication of application: 24.12.2008
(73) Proprietor: Taylor, Jean, 06400 Cannes (FR)
(72) Inventor: Taylor, Jean, 06400 Cannes (FR)
(74) Representative: Harrison Goddard Foote
(86) International application number: PCT/IB2007/000982
(87) International publication number: WO 2007/119157

(56) References cited:
- WO-A-99/42051
- FR-A- 2 717 675
- FR-A- 2 730 156
- FR-A- 2 844 179
- FR-A1- 2 851 154
- US-A- 5 496 318

## Description

This invention relates to a device for treating vertebrae, including an interspinous implant.

In the document No. WO 99 42051, in the name of the applicant, it is known to produce a flexible interspinous implant, capable of cushioning the posterior movement of the vertebrae during extension of the spinal column. This implant is substantially in the shape of an "H," i.e., it includes an upper recess and a lower recess capable of receiving the spinous processes of the two vertebrae; it is formed from a block of flexible material, made of silicone in particular, possibly contained inside a casing made of a woven material, made of polyester in particular.

This implant is satisfactory in its usage, constituting an alternative to the other, more invasive methods for treating vertebrae, in particular the interpedicular osteosynthesis methods consisting of installing a rigid device with pedicular screws and connecting rods joining together the treated vertebrae.

However, it appears that, in some cases, particularly in the case of patients who are strong or have localised compensatory hyperlordosis, the existing implant has an insufficient cushioning capability. Installation in such patients of the larger size implant existing within the range of available implants is not a satisfactory solution since it is liable to cause too large of a distraction of the vertebrae, leading to excessive anterior disc pressure.

The documents US 5 496 318, FR 2 844179 and FR 2 730 156 disclose various other interspinous implant systems, which do not however make it possible to remedy the above-mentioned problem. In particular, the document US 5 496 318 discloses an implant that is necessarily rigid, taking into consideration the connecting sections of the two portions of the implant (the recommended material for the implant can in particular be high-density polyethylene).

FR 2 851 154 discloses a spinal attachment for damping movements of adjacent vertebrae comprising two cushions with inner surfaces held against surfaces of spiny processes. Each cushion comprises a transverse perforation in which a ligament goes through for ensuring that the cushions may be attached to spinous processes of the vertebrae.

This invention aims to remedy the aforesaid problem of the insufficient cushioning capability of the implant.

In a manner known per se, the device in question includes an implant having a flexible structure, capable of being inserted between the spinous processes of two vertebrae, the areas of these respective spinous processes bearing against the implant defining a longitudinal axis of the implant.

According to the invention, the device includes at least one cerclage capable of being adjustably engaged around the implant, at a centre section of this implant, in a plane perpendicular to said longitudinal axis; this cerclage is made of a material that is inextensible in the circumferential direction of this cerclage, so that it makes it possible to restrain deformation of the implant at the centre section, within the entire said plane, during exertion on this centre section of opposing compressive forces exerted by the spinous processes of the vertebrae during a posterior movement of the spinal column.

As at matter of fact, in certain clinical anatomic situations, the inventor was able to observe an insufficient cushioning capability of the existing implant, resulting from a lateral creep of the implant material under the effect of said compressive forces. The cerclage according to the invention makes it possible to impede this lateral deformation and to thus preserve a sufficient cushioning capability at the centre section of the implant, even in the case of strong compressive forces, such as may exist in patients who are strong or suffering from hyperlordosis.

This cerclage can be put into place before inserting the implant into the patient, or can be put into place on the implant after the latter has been placed on the spinal column of the patient. In the second case, the implant retains a significant degree of flexibility, enabling it to be inserted and put into place in a minimally invasive manner.

The cerclage could have a reduced width, e.g., a circular cross-section. Preferably, however, the cerclage has a flattened cross-section forming a planar surface for bearing against the implant.

The risk of a shearing effect of the implant material on either side of the cerclage is thereby greatly reduced, and the bearing surface of the cerclage against the implant is better distributed.

In the same aim, the cerclage may include rounded edges on said planar surface for bearing.

The cerclage preferably includes an increased width near at least one of its portions intended to be applied against the lateral areas of the implant.

This width increase makes it possible to strengthen the restraining function of the cerclage and to increase the contact surface between the cerclage and the implant, in order to eliminate any shearing risk of the implant material.

Alternatively, and for the same purpose, the device can include at least one bearing plate made of a rigid and heavy-duty material, intended to be inserted between the implant and the cerclage.

Said increased width portion of the cerclage, or said plate, can have a dimension such that it is capable of widely wrapping around a corresponding lateral area of the implant, i.e., of extending not only over this lateral area but also partially over the posterior and anterior faces of the implant.

This wrap-around shape furthers the lateral restraint of the implant. In the case of said plate, it also enables a certain degree of retention of this plate on the implant, facilitating the installation of the device.

On its outside face, at least one plate advantageously includes a recess for receiving the cerclage.

This recess enables the cerclage to be wedged in relation to the plate, ensuing their positioning in relation to each other and preventing a risk of the cerclage slipping over the course of time, in relation to the implant.

The plate and/or the cerclage can have shapes enabling assembly of this plate and this cerclage, in particular by snapping or force-fitting together, with a self-locking effect.

This assembly contributes to facilitating the installation of the cerclage and the plate or plates on the implant.

The cerclage can consist of a continuous strip of material inserted over the implant, or can consist of a strip of material whose ends are joined together after installation. In this case, these ends can be joined together by any means, in particular by tying, welding, suturing, with a non-return cam of the type described in the document WO 94 26192, by crimping, or by deforming and/or folding the material.

The invention will be well understood, and other characteristics and advantages of it will become apparent, with reference to the appended schematic drawing, which, for non-limiting illustrative purposes, shows several possible embodiments of the device to which it relates.
Figure 1 is a posterior view of an existing implant, the spinous processes of the vertebrae being shown as a sectional view and in the lordosis position;
figure 2 is a view similar to figure 1, the spinous processes of the vertebrae being shown in extended position of the spinal column, i.e., exerting opposing pressures on the implant;
figure 3 is a perspective view of the device according to the invention, according to one embodiment;
figure 4 is a view of this device according to another embodiment, as a sectional view of the centre section of an implant that includes this device;
figure 5 is a perspective view of the device, similar to figure 2, and
figure 6 is an enlarged-scale detail view of another embodiment of the device.

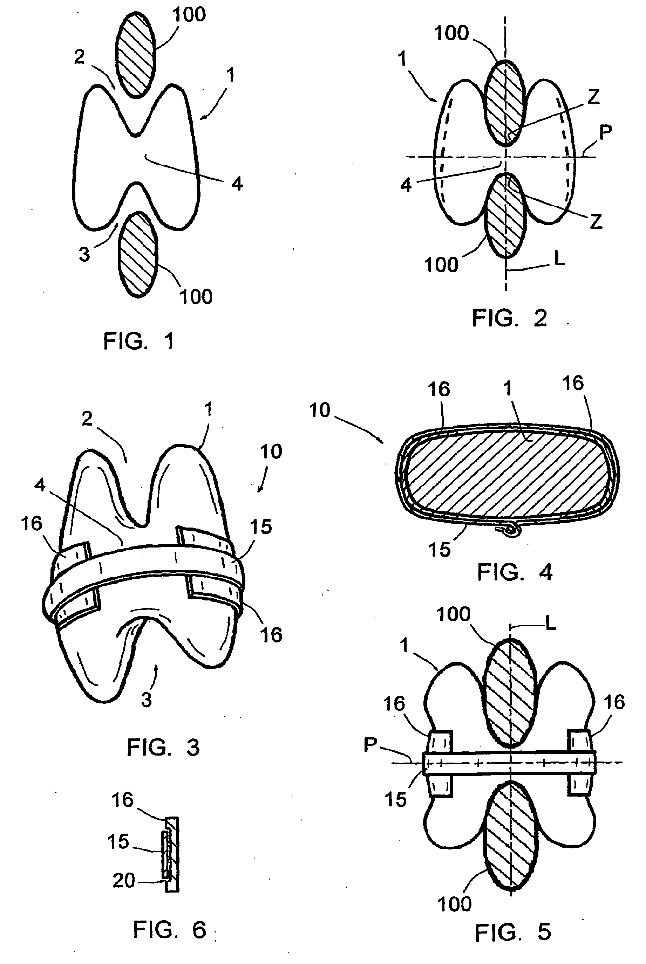

Figures 1 and 2 show an existing flexible interspinous implant 1, capable of being inserted between the spinous processes 100 of two vertebrae so as to be able to cushion the posterior movement of these vertebrae during extension of the spinal column. The implant 1 is substantially in the shape of an "H," i.e., it includes an upper recess 2 and a lower recess 3 capable of receiving the spinous processes 100; it is formed from a block of flexible material, made of silicone in particular, possibly contained inside a casing made of a woven material, made of polyester in particular.

In the event of a posterior movement of the spinal column, as is shown in figure 2, the spinous processes 100 exert opposing pressures on the centre section 4 of the implant 1, which can be significant in patients who are strong or who have localised compensatory hyperlordosis. Such pressures produce a lateral creep of the implant 1 material, resulting in a thinning of the centre section 4 that does not enable a sufficient capability for cushioning the posterior movements of the vertebrae.

The areas Z of the respective spinous processes 100 bearing against the implant 1 define a longitudinal axis L of the implant 1, and a plane P perpendicular to this longitudinal axis.

Figures 3 to 6 show a device 10 for treating vertebrae, including an interspinous implant 1 such as the one cited above. The device further includes a restraining cerclage 15 and two lateral bearing distribution plates 16.

The cerclage 15 is made of a stretchable material, in particular a synthetic or metallic material. It has a flattened cross-section forming a planar surface for bearing against the implant 1.

As shown in figures 3 to 5, this cerclage 15 is intended to be adjustably engaged around said centre section 4 of the implant 1, in a plane perpendicular to the longitudinal median plane of the implant, i.e., the plane of symmetry of the implant 1 passing through the bottoms of the recesses 2, 3. The cerclage 15 can consist of a continuous strip, as shown in figures 3 or 5, and is put into place before inserting the implant 1 into the body of the patient, or can be put into place on the implant 1 after the latter has been placed on the spinal column of the patient. In the this second case, the cerclage 15 can, in particular, be made of a metallic material and, as shown in figure 4, one of its ends can include a hole into which the other of its ends can be inserted, this other end then being folded over itself and crimped in order to ensure closure of the cerclage 15.

Each plate 16 is made of a rigid and heavy-duty material, in particular a synthetic or metallic material. As shown more particularly in figure 4, when seen from a profile view, it has a "C" shape capable of widely wrapping around a corresponding lateral area of the implant 1, i.e., of extending not only over this lateral area but also partially over the posterior and anterior faces of the implant 1.

As seen in the figures, the two plates 16 are intended to be inserted between the implant 1 and the cerclage 15 and function to increase the contact surface between the cerclage 15 and the implant 1.

Figure 6 shows that, on its outside face, at least one plate 16 can include a recess 20 for receiving the cerclage 15, making it possible to wedge the cerclage 15 in relation to the plate 16. This wedging ensures the positioning of the cerclages 15 and the plate 16 in relation to each other and prevents a risk of this cerclage 15 slipping over the course of time, in relation to the implant 1.

As can be understood with reference to figure 5, and by comparison with figure 2, during the posterior movement of the spinal column, leading to a drawing together of the spinal processes 100 of the two vertebrae treated, the cerclage 15 makes it possible to restrain the lateral deformation of the implant 1 at the centre section 4. This restraint makes it possible to impede the lateral deformation that the implant 1 would otherwise experience, and to thus preserve a sufficient cushioning capability at the centre section 4, even in the case of strong compressive forces, such as can those that can exist in patients who are strong or who suffer from hyperlordosis.

The plates 16 strengthen the restraining function of the cerclage 15 and make it possible to increase the contact surface between the cerclage 15 and the implant 1, in order to eliminate any sharing risk of the implant material. Their wrap-around shapes further the lateral restraint of the implant 1 and enable a certain degree of retention of these plates 16 on the implant 1, facilitating the installation of the device.

As is apparent from the preceding, the invention provides a device for treating vertebrae whose interspinous implant has a sufficient cushioning capability in every instance, in particular in the case of patients who are large or who have localized compensatory hyperlordosis.

It is obvious that the invention is not limited to the embodiment described above for illustrative purposes, but that it extends to any embodiment covered by the appended claims.

## Claims

1. Device (10) for treating vertebrae including an implant (1) having a flexible structure, capable of being inserted between the spinous processes (100) of two vertebrae, the areas (Z) of these respective spinous processes (100) bearing against the implant (1) defining a longitudinal axis (L) of the implant, **characterized in that** it includes at least one cerclage (15) capable of being adjustably engaged around the implant (1), at a centre section (4) of this implant (1), in a plane (P) perpendicular to said longitudinal axis (L); this cerclage (15) is made of a material that is inextensible in the circumferential direction of this cerclage (15), so that it makes it possible to restrain deformation of the implant (1) at the centre section (4), within the entire said plane (P), during exertion on this centre section of opposing compressive forces exerted by the spinous processes of the vertebrae during a posterior movement of the spinal column.

2. Device (10) according to claim 1, **characterized in that** the cerclage (15) has a flattened cross-section forming a planar surface for bearing against the implant.

3. Device (10) according to claim 2, **characterized in that** the cerclage (15) incudes rounded edges on said planar surface for bearing.

4. Device (10) according to anyone of claims 1 to 3, **characterized in that** the cerclage (15) includes an increased width near at least one of its portions intended to be applied against the lateral areas of the implant (1).

5. Device (10) according to anyone of claims 1 to 3, **characterized in that** it incudes one bearing plate (16) made of a rigid and heavy-duty material, intended to be inserted between the implant (1) and the cerclage (15).

6. Device (10) according to claims 4 or claim 5, **characterized in that** said increased width portion of the cerclage (15), or said plate (16), can have a dimension such that it is capable of widely wrapping around a corresponding lateral area of the implant (1), i.e., of extending not only over this lateral area but also partially over the posterior and anterior faces of the implant (1).

7. Device (10) according to claim 5 or claim 6, **characterized in that** at least one plate (16) includes on its outside face a recess for receiving the cerclage (15).

8. Device (10) according to anyone of claims 5 to 7, **characterized in that** the plate (16) and/or the cerclage (15) have shapes enabling assembly of this plate (16) and this cerclage (15), in particular by snapping or force-fitting together, with a self-locking effect.

9. Device (10) according to anyone of claims 1 to 8, **characterized in that** the cerclage (115) consists of a continuous strip of material inserted over the implant (1).

10. Device (10) according to anyone of claims 1 to 8, **characterized in that** the cerclage (15) consists of a strip of material whose ends are joined together after installation.

## Patentansprüche

1. Vorrichtung (10) zur Behandlung von Wirbeln, mit einem Implantat (1), das eine flexible Struktur aufweist, wobei die Vorrichtung zwischen die Dornfortsätze (100) von zwei Wirbeln eingeführt werden kann, wobei die Flächen (Z) dieser entsprechenden Dornfortsätze (100) an dem Implantat (1) lagern und eine Längsachse (L) des Implantats definieren, **dadurch gekennzeichnet, dass** die Vorrichtung mindestens eine Cerclage (15) aufweist, die einen anpassbaren Eingriff um das Implantat (1) an einem Mittelabschnitt (4) des Implantats (1) in einer Ebene (P) herstellen kann, die senkrecht zu der genannten Längsachse (L) ist; wobei die Cerclage (15) aus einem Material besteht, das in die umfängliche Richtung der Cerclage (15) nicht ausdehnbar ist, so dass es möglich ist, eine Verformung des Implantats (1) an dem Mittelabschnitt (4) innerhalb der genannten Ebene (P) zu verhindern, während auf diesen Mittelabschnitt entgegengesetzt wirkende Kompressionskräfte wirken, die durch die Dornfortsätze der Wirbel während einer posterioren Bewebung der Wirbelsäule ausgeübt werden.

2. Vorrichtung (10) nach Anspruch 1, **dadurch gekennzeichnet, dass** die Cerclage (15) einen abgeflachten Querschnitt aufweist, der eine planare Oberfläche zum Anliegen an dem Implantat bildet.

3. Vorrichtung (10) nach Anspruch 2, **dadurch gekennzeichnet, dass** die Cerclage (15) abgerundete Kanten an der genannten planaren Oberfläche zum Anliegen aufweist.

4. Vorrichtung (10) nach einem der Ansprüche 1 bis 3, **dadurch gekennzeichnet, dass** die Cerclage (15) nahe mindestens einem ihrer Teilstücke, der an die lateralen Bereiche des Implantats (1) geführt werden soll, eine erhöhte Breite aufweist.

5. Vorrichtung (10) nach einem der Ansprüche 1 bis 3, **dadurch gekennzeichnet, dass** diese eine Lagerplatte (16) aufweist, die aus einem steifen und für hohe Anforderungen geeigneten Material besteht, wobei die Platte dazu dient, zwischen dem Implantat (1) und der Cerclage (15) eingeführt zu werden.

6. Vorrichtung (10) nach Anspruch 4 oder Anspruch 5, **dadurch gekennzeichnet, dass** das genannte Teilstück der Cerclage (15) mit vergrößerter Breite oder die genannte Platte (16) eine Abmessung aufweisen kann, so dass das Teilstück oder die Platte sich großteils um einen entsprechenden lateralen Bereich des Implantats (1) wickeln kann, d.h. sich nicht nur über diesen lateralen Bereich erstrecken kann, sonder auch teilweise über die posterioren und anterioren Seiten des Implantats (1).

7. Vorrichtung (10) nach Anspruch 5 oder Anspruch 6, **dadurch gekennzeichnet, dass** mindestens eine Platte (16) an ihrer Außenseite eine Aussparung zur Aufnahme der Cerclage (15) aufweist.

8. Vorrichtung (10) nach einem der Ansprüche 5 bis 7, **dadurch gekennzeichnet, dass** die Platte (16) und/oder die Cerclage (15) Formen aufweisen, welche den Zusammenbau der Platte (16) und der Cerclage (15) ermöglichen, im Besonderen durch gegenseitiges Einschnappen oder kraftschlüssige Verbindung miteinander mit selbstverriegelnder Wirkung.

9. Vorrichtung (10) nach einem der Ansprüche 1 bis 8, **dadurch gekennzeichnet, dass** die Cerclage (15) aus einem Endlosmaterialstreifen besteht, der über das Implantat (1) eingeführt wird.

10. Vorrichtung (10) nach einem der Ansprüche 1 bis 8, **dadurch gekennzeichnet, dass** die Cerclage (15) aus einem Materialstreifen besteht, dessen Enden nach dem Einbau miteinander verbunden werden.

## Revendications

1. Appareil (10) pour traiter les vertèbres comprenant un implant (1) ayant une structure souple, pouvant être inséré entre les apophyses épineuses (100) de deux vertèbres, les zones (Z) de ces apophyses épineuses respectives (100) appuyant contre l'implant (1) en définissant un axe longitudinal (L) de l'implant, **caractérisé en ce qu'**il comprend au moins un cerclage (15) pouvant venir en prise de façon réglable autour de l'implant (1), au niveau d'une section centrale (4) de cet implant (1), sur un plan (P) perpendiculaire audit axe longitudinal (L) ; ce cerclage (15) est fait d'un matériau qui est inextensible dans la direction circonférentielle de ce cerclage (15), de sorte qu'il permet de limiter la déformation de l'implant (1) au niveau de la section centrale (4), à l'intérieur dudit plan entier (P), pendant l'exertion sur cette section centrale de forces compressives opposées exercées par les apophyses épineuses des vertèbres pendant un mouvement postérieur de la colonne vertébrale.

2. Appareil (10) selon la revendication 1, **caractérisé en ce que** le cerclage (15) a une section transversale aplatie formant une surface planaire pour appuyer contre l'implant.

3. Appareil (10) selon la revendication 2, **caractérisé en ce que** le cerclage (15) comprend des bords arrondis sur ladite surface planaire à des fins d'appui.

4. Appareil (10) selon l'une quelconque des revendications 1 à 3, **caractérisé en ce que** le cerclage (15) comprend une largeur accrue à proximité d'au moins l'une de ses parties destinées à être appliquées contre les zones latérales de l'implant (1).

5. Appareil (10) selon l'une quelconque des revendications 1 à 3, **caractérisé en ce qu'**il comprend une plaque d'appui (16) faite en un matériau rigide et résistant, destinée à être insérée entre l'implant (1) et le cerclage (15).

6. Appareil (10) selon la revendications 4 ou 5, **caractérisé en ce que** ladite partie à largeur accrue du cerclage (15), ou ladite plaque (16), peut avoir une dimension telle qu'elle peut s'enrouler largement autour d'une zone latérale correspondante de l'implant (1), c'est-à-dire de s'étendre non seulement sur cette zone latérale, mais également en partie sur les faces postérieure et antérieure de l'implant (1).

7. Appareil (10) selon la revendication 5 ou 6, **caractérisé en ce qu'**au moins une plaque (16) comprend sur sa face extérieure un retrait pour recevoir le cerclage (15).

8. Appareil (10) selon l'une quelconque des revendications 5 à 7, **caractérisé en ce que** la plaque (16) et/ou le cerclage (15) ont des formes permettant l'assemblage de cette plaque (16) et de ce cerclage (15), en particulier par encliquetage ou forçage ensemble, avec un effet autobloquant.

9. Appareil (10) selon l'une quelconque des revendications 1 à 8, **caractérisé en ce que** le cerclage (15) consiste en une bande continue de matière insérée sur l'implant (1).

10. Appareil (10) selon l'une quelconque des revendications 1 à 8, **caractérisé en ce que** le cerclage (15) consiste en une bande de matière dont les extrémités sont réunies après installation.
